# EUROPEAN PATENT APPLICATION

(11) **EP 3 885 362 A1**
(43) Date of publication of application: **29.09.2021**
(21) Application number: 19886375.5
(22) Date of filing: 21.11.2019
(51) Int. Cl.: C07K 16/18, C07K 17/00, C12N 15/13, G01N 33/53

(54) **ANTIBODY CONJUGATE**

(30) Priority: 22.11.2018 JP 2018219558
(71) Applicant: Fujirebio Inc., Shinjuku-ku Tokyo 163-0410 (JP)
(72) Inventor: JANNES, Geert, 9052 Gent (BE); DAUWE, Martine, 9052 Gent (BE); ISHII, Yuichi, Tokyo 163-0410 (JP); OKADA, Ryo, Tokyo 163-0410 (JP)
(74) Representative: Hoffmann Eitle
(86) International application number: PCT/JP2019/045589
(87) International publication number: WO 2020/105700

(57) **Abstract**

The present invention provides an improvement of sensitivity in immunological measurement of tau protein. Specifically, the present invention provides an antibody conjugate comprising two or more kinds of anti-tau protein antibodies bound to a carrier.

## Description

### TECHNICAL FIELD

The present invention relates to an antibody conjugate and the like.

### BACKGROUND ART

Alzheimer disease (AD) is one of the most common dementia, and is a neurodegenerative disease histologically characterized by changes of neurofibrils in nerve cells across cerebral cortex and cerebral limbic system and extracellular accumulation of amyloid plaques. Ultramicromorphology of neurofibril changes is mainly composed of paired helical filaments (PHF) consisting of aberrantly phosphorylated tau proteins (pTau). Usefulness of tau protein (including phosphorylated tau protein) as a diagnostic marker for the neurodegenerative diseases such as Alzheimer disease and immunological methods for measuring tau protein in body fluid are reported (Patent Literatures 1 and 2).

### PRIOR ART REFERENCES

### PATENT LITERARURES

Patent Literature 1: Japanese Patent Application Publication No. Hei-8-502898
Patent Literature 2: Japanese Patent Application Publication No. Hei-9-506771

### SUMMARY OF INVENTION

### PROBLEM TO BE SOLVED BY THE INVENTION

It is required to further improve sensitivity compared to conventional levels in an immunological measurement of tau protein in a sample such as body fluid.

### MEANS FOR SOLVING PROBLEM

As a result of an extensive study, the present inventors have found that by using an antibody conjugate comprising two or more kinds of anti-tau protein antibodies bound to a carrier, detection sensitivity of tau protein is improved compared with a case of using a single anti-tau protein antibody, and completed the present invention.

That is, the present invention is as follows.
[1] An antibody conjugate comprising two or more kinds of anti-tau protein antibodies bound to a carrier.
[2] The antibody conjugate of [1], wherein the two or more kinds of anti-tau protein antibodies are two or more kinds of anti-human tau protein antibodies.
[3] The antibody conjugate of [2], wherein the two or more kinds of anti-tau protein antibodies comprise a first antibody that recognizes an epitope present between positions 153 and 169 in the amino acid sequence of SEQ ID NO:1 and a second antibody that recognizes an epitope present between positions 188 and 207 in the amino acid sequence of SEQ ID NO:1.
[4] The antibody conjugate of [3], wherein the eptitope recognized by the first antibody is PPGQK (SEQ ID NO:2) and the epitope recognized by the second antibody is DRSGYS (SEQ ID NO:3).
[5] The antibody conjugate of any of [1] to [4], wherein the carrier is a labeled carrier.
[6] A kit comprising:
   (i) an antibody conjugate comprising two or more kinds of anti-tau protein antibodies bound to a carrier; and
   (ii) an anti-tau protein antibody that recognizes an epitope different from epitopes recognized by the anti-tau protein antibodies comprised in the antibody conjugate.
[7] The kit of [6], wherein said different epitope is an epitope present between positions 170 and 187 or between positions 209 and 233 in the amino acid sequence of SEQ ID NO:1.
[8] The kit of [7], wherein said different epitope is PPAPKTP (SEQ ID NO:4) or PPTREPK (SEQ ID NO:5).
[9] A method for detecting tau protein, comprising detecting the tau protein in a sample using an antibody conjugate comprising two or more kinds of anti-tau protein antibodies bound to a carrier.
[10] The method of [9], wherein the sample is a body fluid sample collected from a human being.
[11] The method of [9] or [10], further comprising contacting the sample with an anti-tau protein antibody that recognizes an epitope different from epitopes recognized by the anti-tau protein antibodies comprised in the antibody conjugate.

### EFFECT OF THE INVENTION

By using the antibody conjugate of the present invention, the detection sensitivity of tau protein is improved compared with a case of using a single anti-tau protein antibody in immunological measurements such as a sandwich immunoassay. The detection of tau protein using the antibody conjugate of the present invention is useful for diagnosis with high accuracy for neurodegenerative diseases such as Alzheimer diseases.

### BRIEF DESCRIPTION OF DRAWINGS

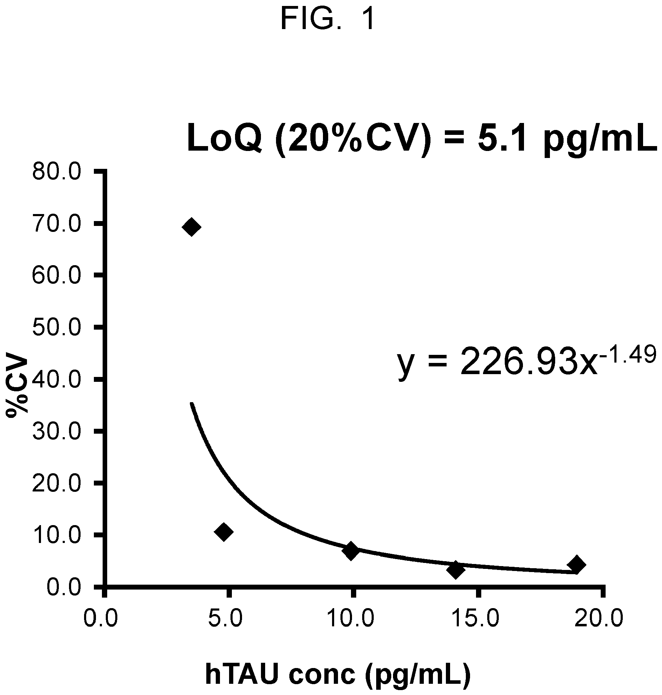

Fig. 1 shows a graph plotting the concentration of each sample (LoQ1 to LoQ5) calculated based on a standard curve prepared from an average count value and a concentration of each standard solution to a coefficient of variance (CV) in count values upon measuring each sample (LoQ1 to LoQ5) ten times (n=10) in the measurement of total tau proteins using a hybrid conjugate, as well as a limit value of quantification (LoQ (CV20%)) obtained based on precision profile.

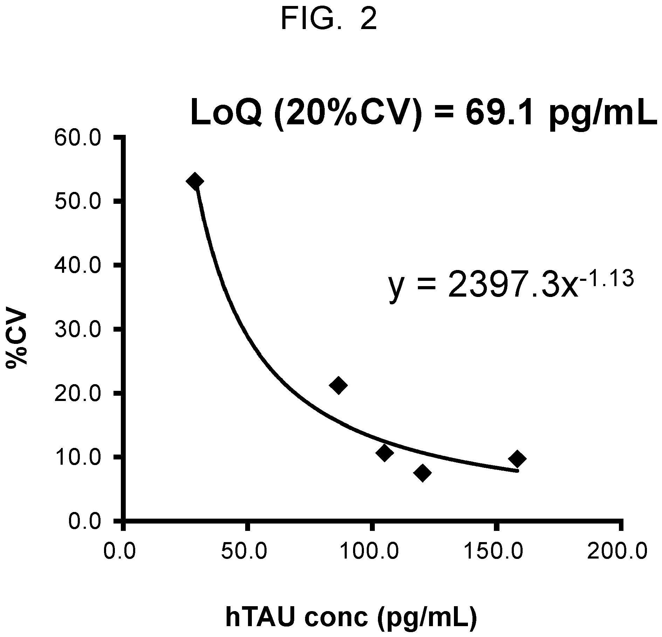

Fig. 2 shows a graph plotting the concentration of each sample (LoQ1 to LoQ5) calculated based on a standard curve prepared from an average count value and a concentration of each standard solution to a coefficient of variance (CV) in count values upon measuring each sample (LoQ1 to LoQ5) ten times (n=10) in the measurement of total tau proteins using an antibody mixture, as well as a limit value of quantification (LoQ (CV20%)) obtained based on precision profile.

### EMBODIMENTS FOR CARRYING OUT THE INVENTION

The present invention provides an antibody conjugate (also referred to as a "hybrid conjugate") with two or more kinds of anti-tau protein antibodies bound to a carrier.

The tau protein is preferably human tau protein. It is known that human tau protein includes a plurality of isoforms, 4 to 6 isoforms are detected in adult brain and only one isoform is detected in embryonic brain. This diversity of the isoforms occurs due to alternative mRNA splicing from one gene present on the human 17th chromosome. These isoforms are different each other in 3 or 4 repeat domains in the C terminal portion and in presence or absence of an insert consisting of 29 or 58 amino acid residues in the N terminal portion. An amino acid number herein in the tau protein is represented as an amino acid number corresponding to the longest isoform (441 amino acids) shown in SEQ ID NO:1. The tau protein includes modified tau proteins. The modified tau protein includes, for example, a phosphorylated tau protein. The phosphorylated tau protein refers to tau protein where a hydroxy group-containing amino acid residue (serine, threonine and tyrosine residues) has been phosphorylated.

The anti-tau protein antibody is an antibody that recognizes at least a portion of an amino acid sequence of the tau protein as an epitope. Two or more kinds of anti-tau protein antibodies may be, for example, 2 to 5, preferably 2 to 4, more preferably 2 to 3, and particularly preferably 2 anti-tau protein antibodies. Two or more kinds of anti-tau protein antibodies each preferably recognize an epitope that is not overlapped in the tau protein.

The two or more kinds of anti-tau protein antibodies comprise a first antibody and a second antibody. The epitope recognized by the first antibody may be an epitope present preferably between positions 153 and 169, more preferably between positions 155 and 167, still more preferably between positions 157 and 165, and may particularly preferably be PPGQK (between positions 159 and 163; SEQ ID NO:2) in the amino acid sequence of SEQ ID NO:1. The epitope recognized by the second antibody may be an epitope present preferably between positions 188 and 207, more preferably between positions 190 and 204, still more preferably between positions 192 and 201, and may particularly preferably be DRSGYS (between positions 193 and 198; SEQ ID NO:3) in the amino acid sequence of SEQ ID NO:1.

The number of amino acid residues present between the epitope recognized by the first antibody and the epitope recognized by the second antibody may be, for example, 18 or more amino acids, preferably 20 or more amino acids, more preferably 22 or more amino acids, still more preferably 24 or more amino acids, and particularly preferably 26 or more amino acids. The amino acid residues present between the two epitopes may be, for example, 44 or less amino acids, preferably 41 or less amino acids, more preferably 38 or less amino acids, still more preferably 35 or less amino acids, and particularly preferably 33 or less amino acids. More specifically, the amino acid residues present between the two epitope may be, for example, 18 to 44 amino acids, preferably 20 to 41 amino acids, more preferably 22 to 38 amino acids, still more preferably 24 to 35 amino acids, and particularly preferably 26 to 33 amino acids.

The epitopes recognized by the two anti-tau protein antibodies may be any of unmodified peptides or modified peptides. The modified peptide includes, for example, phosphorylated peptides. The phosphorylated tau protein refers to a peptide where a hydroxy group-containing amino acid residue (serine, threonine and tyrosine residues) has been phosphorylated.

The anti-tau protein antibody may be either a polyclonal antibody or a monoclonal antibody. The anti-tau protein antibody may be any isotype of an immunoglobulin (e.g., IgG, IgM, IgA, IgD, IgE, IgY). The anti-tau protein antibody may be a full-length antibody. The full-length antibody refers to an antibody comprising heavy chains and light chains each comprising a variable region and a constant region (e.g., an antibody comprising two Fab portions and an Fc portion). The anti-tau protein antibody may also be an antibody fragment derived from the full-length antibody. The antibody fragment is a part of the full-length antibody, and includes, for example antibodies deleting the constant regions (e.g., F(ab')₂, Fab', Fab, Fv). The anti-tau protein antibody may also be a modified antibody such as a single chain antibody.

The anti-tau protein antibody can be produced using a known method. For example, the anti-tau protein antibody can be produced using the above epitope as an antigen. Also, many anti-tau protein antibodies that recognize the epitope as described above are commercially available, and thus, such a commercially available antibody can also be used.

The carrier may be a linker compound or a solid phase. The linker compound refers to a compound having an ability to bind to a plurality of antibodies (e.g., full-length antibody, antibody fragment). The linker compound may be a linker compound having an ability to bind to a label, a linker compound bound to a label, or a linker compound that is a label in itself. The linker compound includes, for example, maleimide, hallo acetyl, isothiocyanate, sulfonyl chloride, N-hydroxy succinimide, azide, polysaccharide (e.g., dextran), peptides, polypeptides, proteins (e.g., bovine serum albumin (BSA)), nucleic acids (DNA, RNA), and polyethylene glycol. The solid phase includes, for example, a solid phase capable of being suspended or dispersed in a liquid phase (e.g., solid phase carriers such as particles, beads), and a solid phase capable of accommodating or mounting a liquid phase (e.g., supports such as plates, membranes, test tubes, and containers such as well plates, microchannels, glass capillaries, nanopiller, monolith columns). A solid phase for the carrier is preferably the solid phase capable of being suspended or dispersed in the liquid phase. Materials for the solid phase include, for example, glasses, silica, polymerous compounds (e.g., polystyrene, plastics), metals, and carbon. Non-magnetic materials or magnetic materials may also be used as the materials for the solid phase.

Known methods can be utilized as a method for binding the antibodies to the carrier. Such a method includes, for example, a physical adsorption method, a covalently binding method, a method employing an affinity substance (e.g., biotin, streptavidin), and an ionic binding method. When the covalently binding method is utilized, the antibodies can be bound covalently to the linker compound using, for example, a periodic acid method, a glutaraldehyde method, a maleimide method, or N-hydroxy succinimide method. As a method for binding the antibodies to the solid phase, the same method as the method for binding the antibodies to the linker compound can be utilized.

The antibody conjugate of the present invention may comprise a label. When the antibody conjugate of the present invention comprises the label, the carrier may be a labeled carrier, or the antibodies may be bound to the label, or the both cases may be possible. The labeled carrier refers to a carrier bound to a label or a carrier that is a label in itself. The label includes, for example, enzymes (e.g., peroxidase, alkaline phosphatase, luciferase, β-galactosidase), affinity substances (e.g., one of streptavidin and biotin, one of a sense chain and an antisense chain that are mutually complementary in a nucleic acid), fluorescent substances (e.g., fluorescein, fluorescein isothiocyanate, rhodamine, green fluorescent protein, red fluorescent protein), luminescent substances (e.g., luciferin, aequorin, acridinium ester, tris (2,2'-bipyridyl) ruthenium, luminol), radioactive substances (e.g., ³H, ¹⁴C, ³²P, ³⁵S, ¹²⁵I), and gold colloid. A carrier bound to a label may be, for example, a particle or a bead comprising a fluorescent substance inside thereof (e.g., fluorescent beads). As materials for particles and beads, the same materials as those for the solid phase for the carrier can be used. The carrier that is the label in itself includes, for example, enzymes (e.g., peroxidase, alkaline phosphatase, luciferase, β-galactosidase), affinity substances (e.g., one of streptavidin and biotin, one of a sense chain and an antisense chain that are mutually complementary in a nucleic acid), fluorescent substances (e.g., fluorescein, fluorescein isothiocyanate, rhodamine, green fluorescent protein, red fluorescent protein) and gold colloid. The antibody conjugate comprising the label may be made by binding the label to the antibody conjugate or by binding the antibody to the labeled carrier. Known methods may be used as methods for binding them.

The antibody conjugate of the present invention can be used for detection of tau protein by an immunoassay. Such an immunoassay includes, for example, a direct competition method, an indirect competition method, a sandwich method, a western blotting method, and an immunohistochemical staining method. Such an immunoassay may preferably be a sandwich method. Also such an immunoassay includes a chemiluminescence immunoassay (CLIA) (e.g., chemiluminescence enzyme immunoassay (CLEIA)), a turbidimetric immunoassay (TIA), an enzyme immunoassay (EIA) (e.g., direct competition ELISA, indirect competition ELISA, and sandwich ELISA), a radioimmunoassay (RIA), a latex agglutination reaction assay, a fluorescent immunoassay (FIA), and an immunochromatography method. In the sandwich method, the antibody conjugate of the present invention may be used as a detection antibody or a capture antibody, and may preferably be used as the detection antibody. When the antibody conjugate of the present invention is used as the detection antibody, tau protein can be detected by binding a label to the antibody conjugate and detecting the label bound to the antibody conjugate. Alternatively, when the antibody conjugate where the carrier is a labeled carrier is used as the detection antibody, tau protein can be detected by detecting the label in the antibody conjugate bound to tau protein.

A label can be detected based on a procedure appropriately selected from known techniques depending on a type of the label. When the label is an enzyme, the label can be detected by detecting an enzyme activity using a signal generating substrate (e.g., fluorescent substrates, luminescent substrates, colorimetric substrates). When the label is an affinity substance, the label can be detected by detecting an enzyme or signal generating substance bound to the affinity substance using the enzyme or signal generating substance having an ability to bind to the affinity substance. The enzyme or signal generating substance having an ability to bind to the affinity substance may be an enzyme or signal generating substance bound to a substance having an ability to bind to the affinity substance. When the label is a fluorescent substance, a luminescent substance or a radioactive substance, the label can be detected by detecting a signal generated from these labels.

The antibody conjugate of the present invention may be provided in a form of a composition (e.g., solution). Alternatively, the antibody conjugate of the present invention may be provided in a form of a device (e.g., form where the antibody conjugate is accommodated in the device).

The present invention also provide a kit comprising
(i) an antibody conjugate comprising two or more kinds of anti-tau protein antibodies bound to a carrier; and
(ii) an anti-tau protein antibody that recognizes an epitope different from epitopes recognized by the anti-tau protein antibodies comprised in the antibody conjugate.

The "antibody conjugate comprising two or more kinds of anti-tau protein antibodies bound to the carrier" is as described above.

An epitope recognized by an "anti-tau protein antibody that recognizes an epitope different from epitopes recognized by anti-tau protein antibodies comprised in the antibody conjugate" (hereinafter referred to as a "different anti-tau protein antibody) (hereinafter referred to as a "different epitope") may be, for example, an epitope that does not overlapped with epitopes recognized by the anti-tau protein antibodies comprised in the antibody conjugate, an epitope present preferably between positions 170 and 187 or between positions 209 and 233, more preferably between positions 172 and 185 or between positions 212 and 230, more preferably between positions 174 and 183 or between positions 215 and 227, and may particularly preferably be PPAPKTP (between positions 176 and 182, preferably T represents a phosphorylated threonine residue; SEQ ID NO:4) or PPTREPK (between positions 218 and 224; SEQ ID NO:5) in the amino acid sequence of SEQ ID NO:1. The different epitope may be either a unmodified peptide or a modified peptide. The modified peptide includes, for example, those described above. A modified amino acid residue in the different epitope includes, for example, phosphorylated threonine at position 181 in the amino acid sequence of SEQ ID NO:1. When the different epitope is a modified peptide, the kit of the present invention can be used for detecting a modified tau protein.

The different anti-tau protein antibody may be immobilized to a solid phase. As a method for immobilizing (binding) an antibody to the solid phase, the same method as the method for binding the antibody to the linker compound described above can be utilized.

The kit of the present invention can be utilized in an immunoassay for tau protein. Such an immunoassay includes methods described above, and may preferably be a sandwich method. In the sandwich method, preferably the antibody conjugate and the different anti-tau protein antibody may be used as a detection antibody and a capture antibody, respectively.

The kit of the present invention preferably comprises (i) an antibody conjugate comprising two or more kinds of anti-tau protein antibodies bound to a carrier, and (ii) an anti-tau protein antibody that recognizes an epitope different from epitopes recognized by the anti-tau protein antibodies comprised in the antibody conjugate (different anti-tau protein antibody) in a mutually insulated form. Specifically, each of the antibody conjugate and different anti-tau protein antibody may be contained in a distinct container (e.g., tube, plate). Alternatively, the antibody conjugate and different anti-tau protein antibody may be provided in a form of a composition (e.g., solution). Alternatively, the kit of the present invention may be provided in a form of a device. Specifically, all of the structural components including the antibody conjugate and different anti-tau protein antibody are contained in the device. Alternatively, a part of the structural components may be contained in the device, while the remaining structural components may not necessarily be contained in the device (e.g., form in which they are contained in a different container). In this case, the structural component not contained in the device may be injected into the device upon detecting tau protein.

In preferred embodiments, the kit of the present invention may have a configuration depending on a type of an immunoassay to be employed. For example, when a sandwich method is employed, the kit of the present invention may comprise a label, a diluent (buffer), a substrate that reacts with the label and tau protein standard as optional structural components. Preferably, the antibody conjugate or the different anti-tau protein antibody may be immobilized to a magnetic particle. A specific example of the configuration of the kit of the invention are the antibody conjugate comprising a labeled carrier, the different anti-tau protein antibody immobilized to a solid phase (e.g., magnetic bead, support, container), a substrate that reacts with the label and tau protein preparation.

The present invention also provides a method for detecting tau protein. The method of the present invention comprises detecting the tau protein in a sample using the antibody conjugate comprising two or more kinds of anti-tau protein antibodies bound to a carrier. The antibody conjugate comprising two or more kinds of anti-tau protein antibodies bound to a carrier is as described above.

Detection of the tau protein in the sample may be carried out by an immunoassay. Such an immunoassay includes, for example, those described above, and may preferably be a sandwich method. In the method of the present invention, the antibody conjugate may be used as a detection antibody or a capture antibody, and may preferably be used as the detection antibody.

The detection of tau protein may be carried out by step of treating a sample with an antibody conjugate to allow the antibody conjugate to bind to tau protein in the sample (e.g., step of bringing the sample into contact with the antibody conjugate), and step of detecting the antibody conjugate bound to the tau protein. The detection of the tau protein may further comprise step of eliminating the antibody conjugate that has not been bound to the tau protein (e.g., washing step). The detection of the antibody conjugate bound to the tau protein may be carried out by step of binding a label to the antibody conjugate and step of detecting the label bound to the antibody conjugate. When the antibody conjugate comprises the label, the detection of the antibody conjugate bound to the tau protein may be carried out by step of detecting the label in the antibody conjugate.

The method of the present invention may further comprises contacting a sample with an anti-tau protein antibody that recognizes an epitope different from epitopes recognized by the anti-tau protein antibodies comprised in the antibody conjugate (different anti-tau protein antibody). The "different anti-tau protein antibody" is as described above. In this case, the detection of the tau protein may be carried out by step of treating a sample with the different anti-tau protein antibody to allow tau protein in the sample to bind (trap) to the different anti-tau protein antibody (e.g., step of bringing the sample into contact with the different anti-tau protein antibody), step of treating the tau protein bound to the different anti-tau protein antibody with the antibody conjugate to allow the antibody conjugate to bind to the tau protein bound to the different anti-tau protein antibody (e.g., step of bringing the antibody conjugate into contact with the tau protein bound to the different anti-tau protein antibody), and step of detecting the antibody conjugate bound to the tau protein. The detection of the tau protein may further comprises step of eliminating a free tau protein or different anti-tau protein antibody (B/F separation or washing step) or step of eliminating the antibody conjugate not bound to the tau protein (e.g., washing step) or both steps thereof. The detection of the antibody conjugate bound to the tau protein is as described above.

The sample may be, for example, a liquid sample (e.g., body fluid, preparation), a tissue sample, or a blotting sample. In particular, the sample may be a body fluid sample collected from a human being. The body fluid sample includes, for example, a blood sample (e.g., whole blood, plasma, serum), cerebrospinal fluid, urine and saliva, and may preferably be cerebrospinal fluid, plasma or serum.

The detection of the tau protein includes, for example, evaluation of the presence or absence or the amount of the tau protein, evaluation of the presence or absence or the amount of the modified tau protein (e.g., phosphorylated tau protein), and evaluation of a degree of modification (e.g., phosphorylation) of the tau protein. Based on these evaluations of the tau protein, the neurodegenerative disease such as Alzheimer disease can be diagnosed. The diagnosis of the neurodegenerative disease such as Alzheimer disease based on the evaluation of the tau protein can be carried out based on known techniques (e.g., Japanese Patent Application Publication Nos. Hei-8-502898 and Hei-9-506771).

### EXAMPLES

Hereinafter, the present invention is described with reference to Examples, but the invention is not limited to these Examples.

### Example 1: Production of hybrid conjugate labeled with alkaline phosphatase

A monoclonal antibody BT2 (epitope: DRSGYS (SEQ ID NO:3), manufactured by Fujirebio Europe) that specifically recognized tau protein and pepsin were mixed in citrate buffer (pH 3.5) and the mixture was incubated at 37°C for one hour to perform pepsin digestion. After stopping the reaction, gel filtration purification was performed using Superdex 200 column (manufactured by GE Healthcare Bioscience) to yield a F(ab')₂ fragment. Next, a concentration of the purified F(ab')₂ fragment was determined, and was adjusted to the concentration of 2.5 mg/mL as needed.

A monoclonal antibody HT7 (epitope: PPGQK (SEQ ID NO:2), manufactured by Fujirebio Europe) that specifically recognized tau protein and bromelain were mixed and the mixture was incubated at 37°C for one hour to perform bromelain digestion. After stopping the reaction, gel filtration purification was performed to yield a F(ab')₂ fragment. Next, a concentration of the purified F(ab')₂ fragment was determined, and was adjusted to the concentration of 2.5 mg/mL as needed.

The resulting BT2 F(ab')₂ fragment and HT7 F(ab')₂ fragment were mixed at a weight ratio of 1:1, and 2-mercaptoethylamine (2-MEA) hydrochloride was added thereto. The mixture was incubated at 37°C for 90 minutes to reduce the both fragments (thiolation). Further, desalting was performed to obtain a mixture of the BT2 Fab' fragment and the HT7 Fab' fragment.

On the other hand, desalted alkali phosphatase (ALP) and N-(4-maleimidebutylyloxy)-succinimide (GMBS) were mixed at a molar ratio of 1:10, and the mixture was incubated at 30°C for one hour to perform maleimidation of ALP. After desalting, the mixture of the BT2 Fab' fragment and HT7 Fab' fragment was mixed with maleimidated ALP at a molar ratio of 2:1, and the mixture was incubated at 25°C for one hour to perform a coupling reaction. The coupling reaction was stopped by adding 2-MEA hydrochloride, and the reaction mixture was further incubated at 25°C for 30 minutes. Then, the reaction was quenched by adding 0.5 M iodoacetamide and incubating at 25°C for 30 minutes to yield a conjugate reaction mixture.

The resulting conjugate reaction mixture was desalted, purified, and subsequently applied on HiLoad Superdex 200 column (manufactured by GE Healthcare Bioscience) to perform gel filtration of the antibodies. Among a plurality of peaks at absorbance of 280 nm in obtained fractions, fractions in which two or more Fab's bound to one ALP were pooled to use as a hybrid conjugate. The hybrid conjugate was purified, and subsequently was adjusted to a concentration of 0.1 mg/mL in 0.1 M MES buffer (1 mM MgCl₂, 0.1 mM ZnCl₂, 0.1% NaN₃, 0.1% BSA, pH 6.8).

### Comparative Example 1: Production of single monoclonal antibody labeled with alkali phosphatase

The monoclinal antibody BT2 and pepsin were mixed in the citrate buffer (pH 3.5) and the mixture was incubated at 37°C for one hour to perform the pepsin digestion. After stopping the reaction, gel filtration purification by Superdex 200 column (GE Healthcare Bioscience) was performed to yield a F(ab')₂ fragment. Next, a concentration of the purified F(ab')₂ fragment was determined and adjusted to a concentration of 2.5 mg/mL as needed.

The resulting F(ab')₂ fragment of BT2 was incubated with 2-MEA hydrochloride at 37°C for 90 minutes to reduce and yield a Fab' fragment of BT2.

On the other hand, desalted alkali phosphatase (ALP) and N-(4-maleimidebutylyloxy)-succinimide (GMBS) were mixed at a molar ratio of 1:10, and the mixture was incubated at 30°C for one hour to perform maleimidation of ALP. After desalting, the Fab' fragment of BT2 and maleimidated ALP were mixed at a molar ratio of 1:1, and the mixture was incubated at 25°C for one hour to perform a coupling reaction. The coupling reaction was stopped by adding 2-MEA hydrochloride and incubating at 25°C for 30 minutes to yield a BT2 Fab' fragment labeled with ALP.

The resulting BT2 Fab' fragment labeled with ALP was purified by gel filtration using Superdex 200 column (GE Healthcare Bioscience). Among a plurality of peaks at absorbance of 280 nm in obtained fractions, fractions in which two or more Fab' fragments had been bound to one ALP was pooled with dividing two groups of a smaller molecule side and a larger molecule side. A fraction on the smaller molecule side was used as a first ALP-labeled BT2 Fab' fragment (1:X), and a fraction on the larger molecule side was used as a second ALP-labeled BT2 Fab' fragment (1:XX). Each ALP-labeled BT2 Fab' fragment was purified, and subsequently adjusted to a concentration of 0.05 mg/mL in 0.1 M MES buffer (1 mM MgCl₂, 0.1 mM ZnCl₂, 0.1% NaN₃, 0.1% BSA, pH 6.8).

A first and second ALP-labeled HT7 Fab' fragments were prepared in the same manner as that for the first and second ALP-labeled BT2 Fab' fragments.

A first ALP labeled BT2 Fab' fragment solution and a first ALP labeled HT7 Fab' fragment solution prepared above were mixed at a volume ratio of 1:1 to obtain a first ALP-labeled Fab' fragment mixture. Also, a second ALP labeled BT2 Fab' fragment solution and a second ALP labeled HT7 Fab' fragment solution prepared above were mixed at a volume ratio of 1:1 to obtain a second ALP-labeled Fab' fragment mixture.

### Example 2: Production of antibody-bound magnetic beads

A murine monoclinal antibody AT270 that specifically recognized tau protein in which threonine at position 181 had been phosphorylated (epitope: PPAPKT(p)P (SEQ ID NO:4), T(p) represents a phosphorylated threonine residue) and a murine monoclonal antibody AT120 that specifically recognized the tau protein (epitope: PPTREPK (SEQ ID NO:5)) as solid phase-immobilized antibodies were bound to magnetic ferrite particles, respectively.

Specifically, ethyl(dimethylaminopropyl)carbodiimide (EDC) and N-hydroxy succinimide (NHS) were added to carboxylated magnetic ferrite beads (manufactured by Fujirebio), and the mixture was incubated at 25°C for 30 minutes with gently stirring. After washing, the murine monoclonal antibody AT270 was added thereto, and the mixture was incubated at 25°C for one hour with gently stirring. After the reaction, the reaction was stopped by adding trishydroxymethylaminomethane and incubating at 25°C for 30 minutes with gently stirring. After stopping the reaction, the magnetic ferrite particles were separated from the reaction solution by collecting them with a magnet, and washed with 50 mM Tris buffer (containing 0.15 M NaCl, 3% BSA, pH 7.2) to yield a AT270-bound magnetic particles. The resulting AT270-bound magnetic particles were suspended in 50 mM MOPS buffer at a concentration of 0.25 mg/mL to obtain an AT270-bound magnetic particle suspension.

AT120-bound magnetic particles were produced in the same manner as that for the AT270-bound magnetic particles.

### Example 3: Measurement of phosphorylated tau peptide

A phosphorylated tau peptide antigen in which threonine at position 181 in tau protein had been phosphorylated was diluted using 100 mM Tris buffer to prepare phosphorylated tau standard solutions, each concentration of which was 0, 4.5, 9, 18, 36, 72 or 150 pM.

150 µL of the AT270-bound magnetic particle suspension prepared in Example 2 and 100 µL of the diluted phosphorylated tau standard solution were dispensed in a reaction vessel, and the mixture was stirred followed by being incubated at 37°C for 10 minutes. Subsequently, B/F separation and washing were performed. A specific washing solution for "Lumipulse G (trademark) (Lumipulse G Wash Solution, manufactured by Fujirebio) was used for washing. After washing, 250 µL of a solution in which the hybrid conjugate prepared in Example 1 had been diluted with 50 mM MOPS buffer (containing 1% BSA, pH 6.8) to 0.2 µg/mL was dispensed in the reaction vessel, and the mixture was incubated at 37°C for 10 minutes. Subsequently, B/F separation and washing were performed. After washing, 200 µL of a substrate solution containing 3-(2'-spiroadamantan)-4-methoxy-4-(3"-phosphoryloxy)phenyl-1,2-dioxetane disodium salt(AMPPD) that was a chemiluminescence substrate (Lumipulse G Substrate Solution, manufactured by Fujirebio) was dispensed to the reaction vessel, and the mixture was stirred and sunsequently reacted for 5 minutes. A luminescence amount was measured using a luminometer to obtain a count value. Actual measurement was performed using a fully automatic chemiluminescence enzyme immunoassay system "Lumipulse G" (manufactured by Fujirebio).

Using the ALP-labeled single antibody prepared in Comparative Example 1 (first ALP-labeled Fab' fragment mixture and second ALP-labeled Fab' fragment mixture) as a control in place of the hybrid conjugate solution, the standard solutions were measured and the count values were obtained in the same manner as above.

Results are shown in Table 1. The count value of each phosphorylated tau standard solution is shown as a mean of count values obtained by double measurements. A ratio of the count value at 4.5 pM to the count value at 0 pM (S/N ratio) was obtained respectively. As a result, the first ALP-labeled Fab' fragment mixture exhibited low counts. The second ALP-labeled Fab' fragment mixture exhibited similar count values to those from the hybrid conjugate in the standard solutions only at higher concentrations. Meanwhile, only the hybrid conjugate exhibited very high count values in the standard solution even at lower concentrations. Consequently, it was demonstrated that the ratio of the count value in the standard solution at the lowest concentration (4.5 pM) to the count value in the blank (0 pM) (S/N ratio) was about 6 fold higher in the case of using the hybrid conjugate than in the case of using the ALP-labeled Fab' fragment mixture (ALP-labeled single antibody).

The high S/N ratio is an important factor for obtaining a stable assay system and obtaining a high sensitivity. It was shown that the highly sensitive and stable assay system was able to be constructed by the use of the hybrid conjugate.

**Table 1. Measurement of phosphorylated tau using hybrid conjugate and ALP-labeled single antibody**

| Standard solution antigen concentration (pM) | Count value | | |
|---|---|---|---|
| | Hybrid conjugate | ALP-labeled single antibody | |
| | | First ALP-labeled Fab' fragment mixture | Second ALP-labeled Fab' fragment mixture |
| 0 | 595 | 323 | 725 |
| 4.5 | 42613 | 4176 | 9271 |
| 9 | 73769 | 9206 | 21273 |
| 18 | 152858 | 22904 | 51744 |
| 36 | 297171 | 61908 | 143370 |
| 72 | 576113 | 175976 | 398130 |
| 150 | 1346270 | 527862 | 1225542 |
| S/N (4.5/0) | 71.7 | 12.9 | 12.8 |

### Example 4: Measurement of total tau protein

150 µL of the AT120-bound magnetic bead suspension prepared in Example 2, 75 µL of a calibrator reagent for measuring a total tau protein ("LUMIPULSE G Total Tau Calibrators set" manufactured by Fujirebio) or tau protein standard solution prepared by diluting the calibrator reagent using 50 mM Tris buffer, and 50 µL of a solution prepared by diluting the hybrid conjugate made in Example 1 with 50 mM Tris buffer to a concentration of 0.2 µg/mL were dispensed in a reaction vessel, and the mixture was stirred and then incubated at 37°C for 20 minutes. Subsequently the B/F separation and washing were carried out. After washing, 200 µL of a substrate solution containing AMPPD (Lumipulse G Substrate Solution, manufactured by Fujirebio) was dispensed into the reaction vessel, and the mixture was stirred and then incubated at 37°C for 5 minutes. A luminescence amount was measured using a luminometer to obtain a count value. Actual measurement was performed using the fully automatic chemiluminescence enzyme immunoassay system "Lumipulse G" (manufactured by Fujirebio).

A biotinylated monoclonal antibody BT2 (manufactured by Fujirebio Europe), a biotinylated monoclonal antibody HT7 (manufactured by Fujirebio Europe), and ALP labeled with streptavidin in place of the hybrid conjugate were used as controls to measure standard solutions as follows.

150 µL of AT120-bound magnetic particle suspension prepared in Example 2, 75 µL of the above tau protein standard solution, and 50 µL of a solution containing the biotinylated monoclinal antibody BT2 and biotinylated monoclonal antibody HT7 (antibody mixture) (total antibody 8 µg/mL) were dispensed in a reaction vessel, and the mixture was stirred and then incubated at 37°C for 10 minutes. Subsequently the B/F separation and washing were carried out. After washing, 250 µL of a solution of 0.08 µg/mL ALP labeled with streptavidin (SA-ALP) was dispensed in the reaction vessel, and the mixture was incubated at 37°C for 10 minutes. Subsequently the B/F separation and washing were carried out. After washing, 200 µL of a substrate solution containing AMPPD (Lumipulse G Substrate Solution, manufactured by Fujirebio) was dispensed in the reaction vessel, and the mixture was stirred and then reacted at 37°C for 5 minutes. A luminescence amount was measured using a luminometer to obtain a count value.

Also, samples obtained by diluting a cerebrospinal fluid specimen with the buffer (LoB, LoQ1 to were measured in the same manner as above, and count values were obtained.

Results are shown in Table 2. Table 2 shows the measured count values, the S/N ratios, and LoQ (limit of quantification) determined in the both assay systems. The count value in each standard solution (CAL) is represented as a mean of the count values obtained by doubly measuring the standard solution. The count value of the sample is represented as a mean of the count values obtained by measuring samples with n=10. The S/N ratio was obtained as a ratio of the count value at 600 pg/mL to the count value in the blank (0 pg/mL). The count value at 600 pg/mL was calculated from the count values of the standard solutions.

Figure 1 (hybrid conjugate) and Figure 2 (antibody mixture) plot a concentration of each sample (LoQ1 to LoQ5) calculated based on the standard curve prepared from an average count value and a concentration of each standard solution to a coefficient of variance (CV) in count values upon measuring each sample (LoQ1 to LoQ5) with n=10. In Figures 1 and 2, LoQ (CV 20%) for the both assay systems were obtained based on precision profile.

As a result, in regard to the total tau protein, the use of the hybrid conjugate produced much higher count values than the mixture of the labeled antibodies, and the S/N ratio was about 6 fold higher in the case of using the hybrid conjugate than the case of using the mixture of the labeled antibodies. Further, LoQ in the case of using the hybrid conjugate was about 1/10 relative to LoQ in the case of using the mixture of the labeled antibodies. Thus, it was found that the total tau protein at lower concentrations was able to be quantified by the use of the hybrid conjugate.

From the above results, it was shown that by the use of the hybrid conjugate, the highly sensitive and stable assay system was able to be constructed even in the assay system for the total tau protein.

**Table 2. Measurement of total tau protein using hybrid conjugate and antibody mixture**

| Hybrid conjugate | | | Antibody mixture (two-step conjugation) (Conjugation 1:BT2-bio + HT7-bio) (Conjugation 2:SA-ALP) | | |
|---|---|---|---|---|---|
| | pg/mL | Count value | | pg/mL | Count value |
| CAL | 0 | 744 | CAL | 0 | 409 |
| | 101 | 9281 | | 60 | 514 |
| | 213 | 17613 | | 150 | 817 |
| | 667 | 50422 | | 420 | 2672 |
| | 1037 | 83604 | | 900 | 7069 |
| | 2639 | 211131 | | 2250 | 38069 |

| | Sample name | Count value | | Sample name | Count value |
|---|---|---|---|---|---|
| Sample | LoB | 728 | Sample | LoB | 408 |
| | LoQ 1 | 982 | | LoQ 1 | 455 |
| | LoQ 2 | 1159 | | LoQ 2 | 585 |
| | LoQ 3 | 1602 | | LoQ 3 | 641 |
| | LoQ 4 | 1966 | | LoQ 4 | 694 |
| | LoQ 5 | 2385 | | LoQ 5 | 858 |
| S/N | 600/0 | 60.6 | S/N | 600/0 | 10 |
| LoQ (20%CV) | | 5.1 | LoQ (20%CV) | | 69.1 |

### Example 5: Manufacture 1 of bovine serum albumin (BSA) linker hybrid conjugate

Bovine serum albumin (BSA) and GMBS were mixed at a molar ratio of 1:10, and incubated at 30°C for one hour to maleimidate BSA. After desalting, the mixture of the BT2 Fab' fragment and the HT7 Fab' fragment obtained in Example 1 was mixed with maleimidated BSA at a molar ratio of 2:1, and the resulting mixture was incubated at 25°C for one hour to perform a coupling reaction. The coupling reaction was stopped by adding 2-MEA hydrochloride, and the reaction mixture was further incubated at 25°C for 30 minutes. Then, the reaction was quenched by adding 0.5 M iodoacetamide and incubating at 25°C for 30 minutes to obtain a conjugate reaction mixture.

The obtained conjugate reaction mixture was applied onto a Hi Load Superdex 200 column (manufactured by GE Healthcare Bioscience) equilibrated with phosphate buffer (pH 6.8) to purify the antibody by gel filtration. Fractions in which two or more Fab' fragments had been bound to one BAS molecule in a plurality of resulting fractions having a peak at absorbance of 280 nm were pooled to yield a BSA linker hybrid conjugate.

The obtained BSA linker hybrid conjugate was mixed with NHS-PEG4-Biotin (manufactured by Thermo Fisher) at a molar ratio of 1:30, and the mixture was incubated at 25°C for 45 minutes to biotinylate the BSA linker hybrid conjugate. Further a desalting treatment yielded a biotinylated BSA linker hybrid conjugate 1. Further, the biotinylated BSA linker hybrid conjugate 1 was diluted to 0.4 µg/mL with 50 mM MOPS buffer (150 mM sodium chloride, 1.0% BSA, pH 6.8), and streptavidin-conjugated alkaline phosphatase (ALP-SA) (Streptavidin, Alkaline Phosphatase Conjugate manufactured by Invitrogen) was added at a concentration of 0.4 µg/mL thereto to obtain a reaction solution comprising the BSA linker hybrid conjugate 1 labeled with ALP (hybrid conjugate reaction solution 1).

### Example 6: Manufacture 2 of BSA linker hybrid conjugate

2-MEA hydrochloride was added to BSA and the mixture was incubated at 37°C for 60 minutes to reduce BSA (thiolation). The desalting treatment was further given to obtain thiolated BSA. Further, thiolated BSA and NHS-PEG4-Bition (manufactured by Thermo Fisher) were mixed at a molar ratio of 1:25 and thiolated BSA and 1,2-bis(maleimide)ethane (manufactured by Tokyo Chemical Industry) were mixed at a molar ratio of 1:350, and the mixtures were incubated at 30°C for one hour to perform biotinylation and maleimidation of BSA simultaneously. After desalting, the mixture of BT2 Fab' fragment and HT7 Fab' fragment obtained in Example 1 was mixed biotinylated and maleimidated BSA at a molar ratio of 2:1, and the reaction mixture was incubated at 25°C for one hour to perform a coupling reaction. The coupling reaction was stopped by adding 2-MEA hydrochloride, and the reaction mixture was further incubated at 25°C for 30 minutes. Then, the reaction was quenched by adding 0.5 M iodoacetamide and incubating at 25°C for 30 minutes to obtain the biotinylated BSA linker hybrid conjugate 2.

The resulting biotinylated BSA linker hybrid conjugate 2 was diluted to 0.2 µg/mL with 50 mM MOPS buffer (150 mM sodium chloride, 1.0% BSA, pH 6.8), and ALP-SA (Streptavidin, Alkaline Phosphatase Conjugate manufactured by Invitrogen) was added at a concentration of 0.4 µg/mL thereto to obtain a reaction solution comprising the BSA linker hybrid conjugate 2 labeled with ALP (hybrid conjugate reaction solution 2).

### Comparative Example 2: Production of BSA linker single monoclonal antibody

The monoclonal antibody BT2 and pepsin were mixed in citrate buffer (pH 3.5) and the mixture was incubated at 37°C for one hour to perform pepsin digestion. After stopping the reaction, purification by gel filtration was performed using Superdex 200 column (GE Healthcare Bioscience) to obtain a F(ab')₂ fragment. Subsequently, a concentration of the purified F(ab')₂ fragment was determined, and it was adjusted to a concentration at 2.5 mg/mL as needed.

The resulting F(ab')₂ fragment of BT2 was reduced by incubating with 2-MEA hydrochloride at 37°C for 90 minutes to obtain a Fab' fragment of BT2.

On the other hand, desalted BSA and GMBS were mixed at a molar ratio of 1:10 and incubated at 30°C for one hour to maleimidate BSA. After desalting, the Fab' fragment of BT2 and maleimidated BSA were mixed at a molar ratio of 2:1 and incubated at 25°C for one hour to perform a coupling reaction. The coupling reaction was stopped by adding 2-MEA hydrochloride, and the reaction mixture was further incubated at 25°C for 30 minutes. Then, the reaction was quenched by adding 0.5 M iodoacetamide and incubating at 25°C for 30 minutes to obtain a BSA linker BT2 Fab' fragment.

The resulting BSA linker BT2 Fab' fragment was purified by gel filtration using a Superdex 200 column (GE Healthcare Bioscience) equilibrated with phosphate buffer (pH 6.8). Fractions in which two or more Fab' fragments had been bound to one ALP molecule in a plurality of obtained fractions having a peak at absorbance of 280 nm were pooled.

A BSA linker HT7 Fab' fragment was prepared in the same manner as that for the BSA linker BT2 Fab' fragment.

The resulting BSA linker BT2 Fab' fragment and BSA linker HT7 Fab' fragment were diluted to 0.2 µg/mL, respectively with 50 mM MOPS buffer (150 mM sodium chloride, 1.0% BSA, pH 6.8), and ALP-SA (Streptavidin Alkaline Phosphatase Conjugate manufactured by Invitrogen) was added at a concentration of 0.4 µg/mL thereto to obtain a mixture of an ALP-labeled BSA linker BT2 Fab' fragment and an ALP-labeled BSA linker HT7 Fab' fragment (mixed conjugate reaction solution).

### Example 7: Measurement of phosphorylated tau peptide

A phosphorylated tau peptide antigen in which threonine at position 181 in tau protein had been phosphorylated was diluted using 100 mM Tris buffer to prepare phosphorylated tau standard solutions, each concentration of which was 0, 40, 100, 200, 400 pg/mL.

150 µL of the AT270-bound magnetic particle suspension prepared in Example 2 and 30 µL of the diluted phosphorylated tau standard solution were dispensed in a reaction vessel, and the mixture was stirred followed by being incubated at 37°C for 10 minutes. Subsequently, B/F separation and washing were performed. A specific washing solution for "Lumipulse G (Lumipulse G Wash Solution, manufactured by Fujirebio) was used for washing. After washing, 250 µL of the hybrid conjugate reaction solution 1 manufactured in Example 5 or the hybrid conjugate reaction solution 2 manufactured in Example 6 was dispensed in the reaction vessel, and the mixture was incubated at 37°C for 10 minutes. Subsequently, B/F separation and washing were performed. After washing, 200 µL of a substrate solution containing AMPPD (Lumipulse G Substrate Solution, manufactured by Fujirebio) was dispensed to the reaction vessel, and the mixture was stirred and subsequently reacted at 37°C for 5 minutes. A luminescence amount was measured using a luminometer to obtain a count value. Actual measurement was performed using a fully automatic chemiluminescence enzyme immunoassay system "Lumipulse G" (manufactured by Fujirebio).

Using the mixed conjugate reaction solution prepared in Comparative Example 2 in place of the hybrid conjugate reaction solution, the standard solutions were measured and the count values were obtained in the same manner as above.

Results are shown in Table 3. The count value of each phosphorylated tau standard solution is shown as a mean of count values obtained by double measurements. A ratio of the count value at 40 pg/mL to the count value at 0 pg/mL (S/N ratio) was obtained, respectively. As a result, low counts were observed when the mixed conjugate reaction solution was used. On the other hand, when the hybrid conjugate reaction solution 1 and the hybrid conjugate reaction solution 2 were used, very high count values were observed even for the standard solution at low concentrations. Consequently, it was demonstrated that the ratio of the count value in the standard solution at the lowest concentration (40 pg/mL) to the count value in the blank (0 pg/mL) (S/N ratio) was about 9 times higher in the case of using the hybrid conjugate reaction solution 1 than the case of using the mixed conjugate reaction solution (mixture of ALP-labeled single antibodies) and about 13 times higher in the case of the hybrid conjugate reaction solution 2 than in the case of the mixed conjugate reaction solution.

The high S/N ratio is an important factor for obtaining a stable assay system and obtaining a high sensitivity. It was shown that the highly sensitive and stable assay system was able to be constructed by the use of the hybrid conjugate.

**Table 3 Measurement of phosphorylated tau using hybrid conjugate and mixed conjugate of single antibodies**

| Standard solution antibody concentration (pg/mL) | Count value | | |
|---|---|---|---|
| | Hybrid conjugates | | Mixed conjugate (2 kinds of single antibodies) |
| | Hybrid conjugate 1 | Hybrid conjugate 2 | |
| 0 | 312 | 278 | 205 |
| 40 | 3114 | 3966 | 226 |
| 100 | 7394 | 10057 | 259 |
| 200 | 14380 | 19254 | 317 |
| 400 | 28700 | 37283 | 447 |
| S/N (40/0) | 10.0 | 14.3 | 1.1 |

## Claims

1. An antibody conjugate comprising two or more kinds of anti-tau protein antibodies bound to a carrier.

2. The antibody conjugate according to claim 1, wherein the two or more kinds of anti-tau protein antibodies are two or more kinds of anti-human tau protein antibodies.

3. The antibody conjugate according to claim 2, wherein the two or more kinds of anti-tau protein antibodies comprise a first antibody that recognizes an epitope present between positions 153 and 169 in the amino acid sequence of SEQ ID NO:1 and a second antibody that recognizes an epitope present between positions 188 and 207 in the amino acid sequence of SEQ ID NO:1.

4. The antibody conjugate according to claim 3, wherein the epitope recognized by the first antibody is PPGQK (SEQ ID NO:2) and the epitope recognized by the second antibody is DRSGYS (SEQ ID NO:3).

5. The antibody conjugate according to any one of claims 1 to 4, wherein the carrier is a labeled carrier.

6. A kit comprising:
(i) an antibody conjugate comprising two or more kinds of anti-tau protein antibodies bound to a carrier; and
(ii) an anti-tau protein antibody that recognizes an epitope different from epitopes recognized by the anti-tau protein antibodies comprised in the antibody conjugate.

7. The kit according to claim 6, wherein said different epitope is an epitope present between positions 170 and 187 or between positions 209 and 233 in the amino acid sequence of SEQ ID NO:1.

8. The kit according to claim 7, wherein said different epitope is PPAPKTP (SEQ ID NO:4) or PPTREPK (SEQ ID NO:5).

9. A method for detecting tau protein, comprising detecting the tau protein in a sample using an antibody conjugate comprising two or more kinds of anti-tau protein antibodies bound to a carrier.

10. The method according to claim 9, wherein the sample is a body fluid sample collected from a human being.

11. The method according to claim 9 or 10, further comprising contacting the sample with an anti-tau protein antibody that recognizes an epitope different from epitopes recognized by the anti-tau protein antibodies comprised in the antibody conjugate.


SEQUENCE LISTING

<110>  FUJIREBIO INC.

<120>  Antibody conjugate

<130>  RB-R011871

<150>  JP2018-219558
<151>  2018-11-22

<160>  5

<170>  PatentIn version 3.5

<210>  1
<211>  441
<212>  PRT
<213>  Homo sapiens

<400>  1

<210>  2
<211>  5
<212>  PRT
<213>  Homo sapiens

<400>  2

Pro Pro Gly Gln Lys
1               5

<210>  3
<211>  6
<212>  PRT
<213>  Homo sapiens

<400>  3

Asp Arg Ser Gly Tyr Ser
1               5

<210>  4
<211>  7
<212>  PRT
<213>  Homo sapiens

<400>  4

Pro Pro Ala Pro Lys Thr Pro
1               5

<210>  5
<211>  7
<212>  PRT
<213>  Homo sapiens

<400>  5

Pro Pro Thr Arg Glu Pro Lys
1               5